# EUROPEAN PATENT APPLICATION

(11) **EP 4 717 160 A1**
(43) Date of publication of application: **01.04.2026**
(21) Application number: 24811301.1
(22) Date of filing: 02.05.2024
(51) Int. Cl.: A61B 5/055, A61B 5/00, G16H 30/20, G16H 30/40, G06N 3/088

(54) **METHOD AND DEVICE FOR GENERATING PET-MRI FUSION IMAGE BY USING ARTIFICIAL INTELLIGENCE**

(30) Priority: 22.05.2023 KR 20230065514
(71) Applicant: Industry Academic Cooperation Foundation of Yeungnam University, Gyeongsan-si, Gyeongsangbuk-do 38541 (KR)
(72) Inventor: JEON, Ikchan, Daegu 42081 (KR)
(74) Representative: Jung, Minkyu
(86) International application number: PCT/KR2024/005932
(87) International publication number: WO 2024/242362

(57) **Abstract**

The present invention relates to a method for generating a Positron Emission Tomography (PET)-Magnetic Resonance Imaging (MRI) fusion image using artificial intelligence, wherein when an identifier (ID) is entered, MRI images corresponding to the ID of a patient are retrieved to select one MRI image among the MRI images, and a Generative Adversarial Networks (GAN)-based generation model trained to derive a PET-MRI fusion image of the same anatomical position as the MRI image from the MRI image is applied to generate a PET-MRI fusion image of the same anatomical position as the selected MRI image.

## Description

### Technical Field

The present invention relates to a method and an apparatus for generating a Positron Emission Tomography (PET)-MRI fusion image based on a Magnetic Resonance Imaging (MRI) using Artificial Intelligence (AI).

### Background Art

Attempts to combine molecular and high-resolution images have been made for a long time, and PET and MRI are the most widely used imaging equipment in the field of neuroscience research as well as in the clinical field. In particular, it is known that PET and MRI have advantages in confirming the metabolic state and anatomical structure of the lesion, respectively.

In the case of MRI, the metabolic state can be indirectly predicted by the signal intensity of the image, but the performance of PET is much higher than that of MRI in cancer and infectious lesions. Previously, it was common to take PET and MRI and compare them when confirmation of both metabolic and anatomical conditions was required. However, when two tests are performed while moving between test equipment with a time difference, the images obtained from each test cause significant errors in metabolic and anatomical positions due to the time difference and posture change between tests.

On the other hand, an integrated PET-MRI apparatus, which has recently been in the spotlight, is a medical diagnostic imaging apparatus that simultaneously photographs PET and MRI at a place where one lies down to acquire PET and MRI images, and produces PET-MRI fusion images through an image processing process. The integrated PET-MRI apparatus may generate a precise PET-MRI fusion image based on the advantage of minimizing errors that occur when PET and MRI are performed respectively.

However, the integrated PET-MRI has a problem in that it requires higher cost and time compared to the PET or MRI. In addition, there is a problem that such an integrated PET-MRI is difficult to be used universally because it is installed only in some advanced medical institutions so far.

### [Prior Art Document]

### [Patent document]

Korean Patent Registered Publication No. 10-1836322

### Detailed Description of the Invention

### Technical Problem

The present disclosure is designed to solve the above problems, and an object of the present disclosure is to provide a method and apparatus for generating a PET-MRI fusion image based on MRI using AI.

### Technical Solution

According to an aspect of the present disclosure, there is provided a method of generating a PET-MRI fusion image using artificial intelligence, the method including: when a patient identifier (ID) is input, retrieving MRI images corresponding to the patient ID and selecting one MRI image; and applying a conditional generative adversarial neural network (GAN)-based generation model trained to derive a PET-MRI fusion image of the same anatomical position as the MRI image from the MRI image, and generating a PET-MRI fusion image of the same anatomical position as the selected MRI image.

According to an aspect of the present disclosure, there is provided an apparatus for generating a PET-MRI fusion image using artificial intelligence, the apparatus including: an input/output unit configured to, when a patient identifier (ID) is input, call MRI images corresponding to the patient MRI and select one of the ID images; a storage unit configured to store a conditional generative adversarial neural network (GAN)-based generation model trained to derive a PET-MRI fusion image at the same anatomical position as the MRI image from the MRI image; and a processor configured to apply the conditional GAN-based generation model to generate a PET-MRI fusion image at the same anatomical position as the selected MRI image.

### Advantageous Effect

According to one aspect of the present invention described above, the present invention provides a method and an apparatus for generating a PET-MRI fusion image based on MRI using AI, thereby enabling the advantage of PET to be used only with MRI without performing expensive PET, and overcoming the disadvantages of using a contrast agent, a long photographing time, a high examination cost, and radiation exposure during PET-MRI examination.

In addition, compared to expensive and low-accessibility PET, PET-MRI fusion images are generated based on MRI, which is relatively low in cost and has good accessibility, which helps medical staff to judge and has a positive effect on reducing overall medical costs.

### Description of Drawings

FIG. 1 is a diagram illustrating an example of a generation model for generating a PET-MRI fusion image based on conditional GAN according to an embodiment of the present invention,
FIG. 2 is a diagram illustrating an example in which a platform to which a conditional GAN-based generation model is applied generates a PET-MRI fusion image according to an embodiment of the present invention,
FIG. 3 is a flowchart illustrating an operation of generating a PET-MRI fusion image of a platform to which a conditional GAN-based generation model is applied according to an embodiment of the present invention,
FIG. 4 is a flowchart illustrating an operation of generating a PET-MRI fusion image of a platform to which a conditional GAN-based generation model is applied according to another embodiment of the present invention,
FIG. 5 is a diagram illustrating an example of extracting a sagittal plane including a vertebral body from an MRI image according to another embodiment of the present invention, and
FIG. 6 is a block diagram illustrating an apparatus for generating a PET-MRI fusion image according to an embodiment of the present invention.

### Mode for Invention

A detailed description of the present invention, which will be described below, refers to the accompanying drawings, which illustrate specific embodiments in which the present invention may be practiced as examples. These examples are described in detail to be sufficient for those skilled in the art to practice the present invention. It should be understood that the various embodiments of the present invention are different from each other but need not be mutually exclusive. For example, certain shapes, structures, and characteristics described herein may be implemented in other embodiments without departing from the spirit and scope of the present invention with respect to one embodiment. It should also be understood that the position or arrangement of individual components within each disclosed embodiment may be altered without departing from the spirit and scope of the present invention. Accordingly, the detailed description to be described below is not intended to be taken in a limited sense, and the scope of the present invention, if properly described, is limited only by the appended claims along with all the scope equivalent to those claimed by the claims. Similar reference numerals in the drawings refer to the same or similar functions across several aspects.

The components according to the present invention are components defined by functional classification rather than physical classification, and may be defined by functions performed by each. Each component may be implemented as hardware or a program code and a processing unit that perform each function, and functions of two or more components may be included in one component to be implemented. Accordingly, it should be noted that the names given to the components in the following embodiments are not intended to physically distinguish each component, but are given to imply a representative function in which each component is performed, and the technical spirit of the present invention is not limited by the names of the components.

Before describing an embodiment of the present invention, the AI algorithm applied in the present invention will be described.

Synthetic medical data is defined as "non-existent but plausible fake data" made based on actual medical data such as medical information, genomes, and lifelogs, and is generated to protect personal information and confidentiality of data or to apply it to a specific environment that cannot be directly secured. As a representative AI algorithm applied to the generation of synthetic medical data, there is a GAN (Generative Adversarial Networks) algorithm, and unlike the existing deep learning algorithm, it is composed of a generator that generates fake data and a discriminator that distinguishes real data from fake data, so that two opposing systems compete with each other to generate new medical image data.

Hereinafter, exemplary embodiments of the present invention will be described in more detail with reference to the drawings.

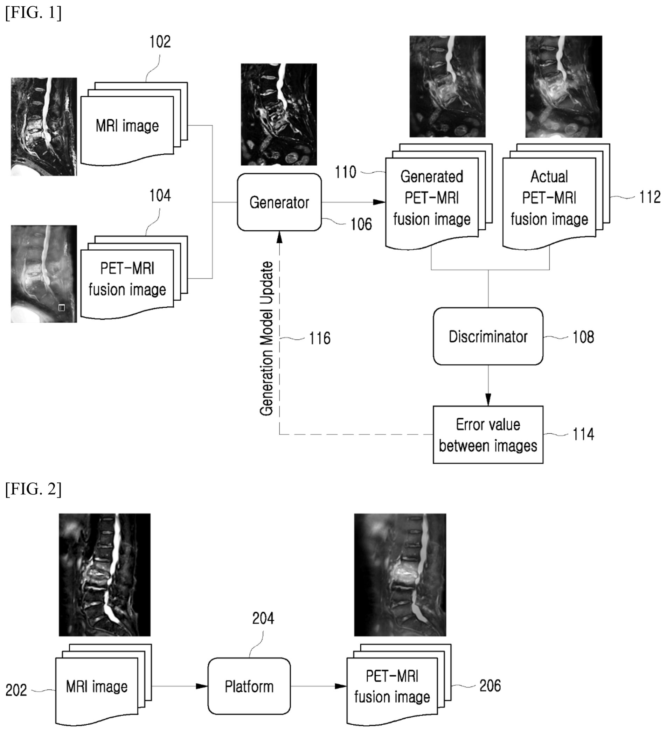

FIG. 1 is a diagram illustrating an example of a generation model for generating a PET-MRI fusion image based on conditional GAN according to an embodiment of the present invention.

Referring to FIG. 1, the generation model according to an embodiment of the present invention is an artificial intelligence model trained to derive a PET-MRI fusion image of the same anatomical position as the MRI image from the MRI image, and the generation model is operated by a generator 106 and a discriminator 108.

In more detail, the generator 106 is trained on MRI images 102 and the PET-MRI fusion image 104 for the same anatomical position secured from the integrated PET-MRI apparatus. In other words, MRI images and PET-MRI fusion images at the same anatomical location for each PET-MRI test are extracted from hundreds of PET-MRI tests and used as training and validation data. The trained MRI image and PET-MRI fusion image are made of pairs, and various sequence images may be used for the MRI image.

The generator 106 that is trained on MRI images 102 and the PET-MRI fusion image 104 for the same anatomical position generates the PET-MRI fusion image of the same anatomical position as the MRI image 102.

The discriminator 108 compares the generated PET-MRI fusion image 110 with the actual PET-MRI fusion image 112 to perform learning to determine authenticity, and repeatedly learns the corresponding process until the similarity between the generated PET-MRI fusion image 110 and the actual PET-MRI fusion image 112 reaches the highest value. Here, the highest value refers to a threshold value used to measure the similarity between the generated PET-MRI fusion image 110 and the actual PET-MRI fusion image 112, and is a value indicating that the similarity between the two images is very high.

In addition, the discriminator 108 measures an error value (loss) between the generated PET-MRI fusion image 110 and the actual PET-MRI fusion image 112 and provides feedback to the generator 106. That is, the discriminator 108 updates the generation model 116 by feeding back the error values between the two images, and through this, the generator 106 may generate the PET-MRI fusion image more similar to the actual PET-MRI fusion image 112.

FIG. 2 is a diagram illustrating an example in which a platform applying a generation model generates a PET-MRI fusion image according to an embodiment of the present invention.

Referring to FIG. 2, it is assumed that a platform 204 operates a conditional GAN-based generation model according to an embodiment of the present invention and various software programs may be executed on the platform.

When the program starts, the platform 204 receives an identifier (ID) of a patient who wants to generate a PET-MRI fusion image from a user, and fetches the MRI images of the patient from a medical image storage of each medical institution and displays it on a screen. In this case, the MRI image displayed on the screen may be configured in the form of a list of MRI images for each sequence.

The user selects the MRI image 202 of a desired sequence from the MRI image list displayed on the screen, and designates a region of interest(ROI) among the selected MRI image 202. Here, a configuration for designating a partial region of interest in the MRI image 202 is not an essential step and may be omitted in some cases.

When the MRI image 202 selected by the user is input, the platform 204 generates the PET-MRI fusion image 206 at the same anatomical position as the MRI image 202 by applying the conditional GAN-based generation model according to an embodiment of the present invention, and displays the generated PET-MRI fusion image 206 on the screen.

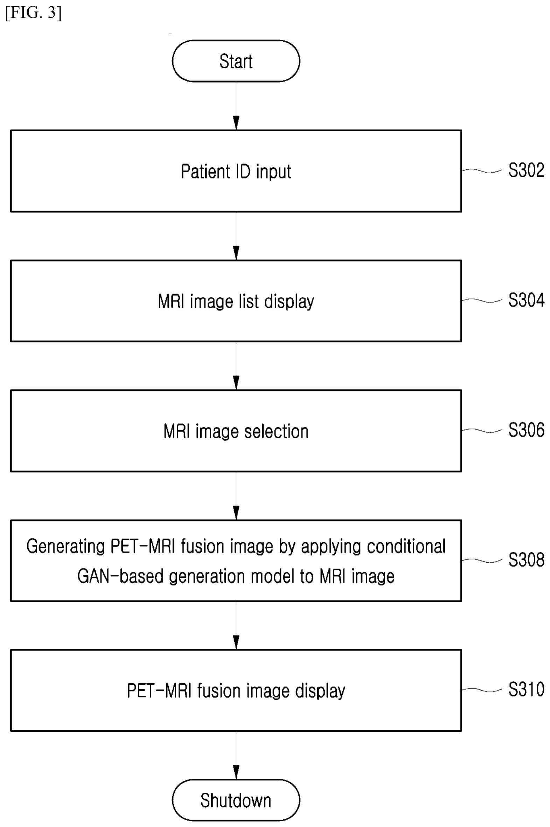

FIG. 3 is a flowchart illustrating an operation of generating a PET-MRI fusion image of a platform to which a conditional GAN-based generation model is applied according to an embodiment of the present invention.

Referring to FIG. 3, when the ID of the patient who wants to generate the PET-MRI fusion image is input through the execution program (S302), the platform fetches the MRI images of the patient from the medical image storage of each medical institution and displays a list of MRI images on the screen. (S304) Here, the MRI image list includes MRI images for various sequences.

When the MRI image is selected from the MRI image list displayed in the S304(S306), a PET-MRI fusion image at the same anatomical position as the selected MRI image is generated by applying the conditional GAN-based generation model according to the embodiment of the present invention to the selected MRI image. (S308)

Thereafter, the platform displays the PET-MRI fusion image generated in step S308 on the screen through the driving program. (S310)

In FIG. 3, an operation of generating a PET-MRI fusion image at the same anatomical position as the entire region of the selected MRI image when the MRI image is selected has been described. However, in order to further improve the performance of the conditional GAN-based generation model according to an embodiment of the present invention, it is possible to extract an MRI image for a partial region of interest from the selected MRI image, and it is also possible to generate a PET-MRI fusion image based on the extracted MRI image.

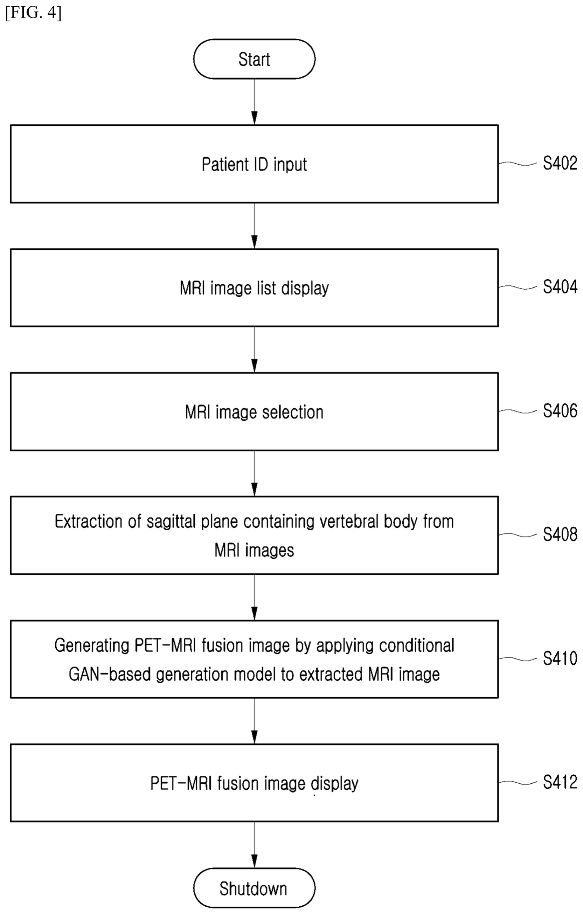

FIG. 4 is a flowchart illustrating a PET-MRI fusion image generation operation of a platform to which a conditional GAN-based generation model is applied according to another embodiment of the present invention.

Referring to FIG. 4, when the ID of the patient who wants to generate the PET-MRI fusion image is input through the execution program (S402), the platform fetches the MRI images of the patient from the medical image storage of each medical institution and displays the MRI image list on the screen. (S404) Here, the MRI image list includes MRI images for various sequences.

When the MRI image is selected from the MRI image list displayed in step S404(S406), a sagittal plane including a region of interest (ROI), for example, the vertebral body, is extracted from the selected MRI image. (S408) Here, it is assumed that the sagittal plane means a virtual plane dividing the body into left and right, and the MRI image selected in S406 is a T2FS (T2 Fat Saturation) sequence MRI image. Here, the T2FS sequence is an MRI sequence designed for fat attenuation, which is a sequence that suppresses high signals in adipose tissue so that signals in other tissues can be seen better.

In addition, the extraction operation of the S408 may be directly designated by the user on the screen to extract the sagittal plane including the vertebral body, and as another example, the sagittal plane including the vertebral body may be automatically extracted through repeated machine learning of the AI model trained to extract the sagittal plane including the vertebral body from the MRI image. In this way, only the sagittal plane including the vertebral body is extracted from the MRI image and used for generating the PET-MRI fusion image because the sagittal plane including various organs other than the vertebrae on the left and right outer sides of the vertebral body may degrade the performance of the generative model according to the embodiment of the present invention.

Thereafter, the platform generates a a PET-MRI fusion image at the same anatomical position as the extracted MRI image by applying a conditional GAN-based generation model according to an embodiment of the present invention to the MRI image extracted from the S408 (S410), and displays the generated PET-MRI fusion image on a screen through a driving program. (S412)

FIG. 4 is a diagram illustrating an example of applying the PET-MRI fusion image generation technology proposed in the present invention to the vertebral body as another embodiment of the present invention. However, the PET-MRI fusion image generation technology proposed in the present invention may be applied to any region of the body according to the process described in FIG. 3. In addition, although it is described in the present specification that PET-MRI image is generated using the T2FS sequence MRI image, other suitable sequence images may also be used according to the body region to which the PET-MRI fusion image generation technology is applied.

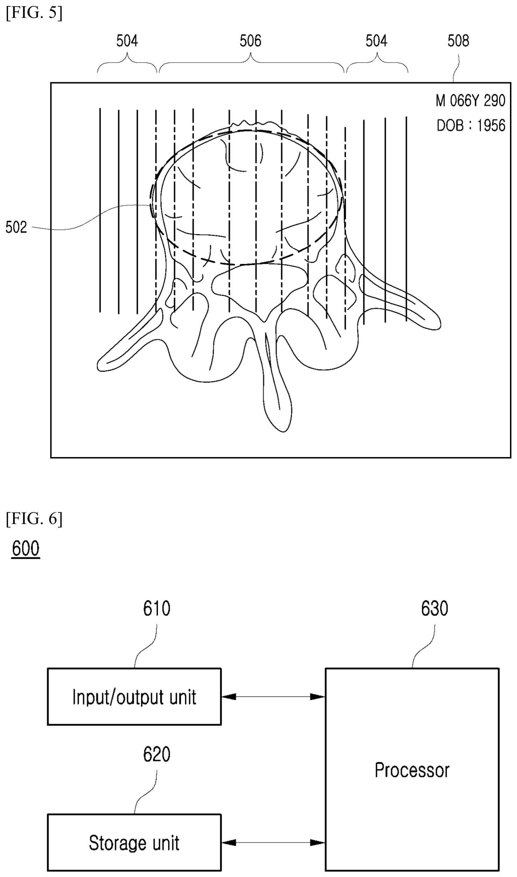

FIG. 5 is a diagram illustrating an example of extracting a sagittal plane including a vertebral trunk bone from an MRI image according to another embodiment of the present invention.

Referring to FIG. 5, only the sagittal plane image 506 including the vertebral body 502 is extracted from the sagittal plane image obtained at the horizontal plane of the spine in the MRI image 508, and the sagittal plane image on the left and right outer sides 504 of the vertebral body 502 is excluded, so that the extracted MRI image used in the embodiment of the present invention may be obtained.

FIG. 6 is a block diagram illustrating an apparatus for generating a PET-MRI fusion image according to an embodiment of the present invention.

Referring to FIG. 6, the PET-MRI fusion image generating apparatus 600 according to an embodiment of the present invention includes an input/output unit 610, a storage unit 620, and a processor 630. In addition, although not shown, the PET-MRI fusion image generating apparatus 600 according to an embodiment of the present invention may further include a communication unit for transmitting and receiving input and output data.

The PET-MRI fusion image generating apparatus 600 receives the ID of the patient, the MRI to be used for generating the PET-MRI fusion image, and other related data from the user through the input/output unit 610, and generates the PET-MRI fusion image by applying the conditional GAN-based generation model proposed in the present disclosure to the MRI input through the processor 630. In addition, the MRI image list of the patient and the generated PET-MRI fusion image are output through the input/output unit 610.

The PET-MRI fusion image generating apparatus 600 stores the conditional GAN-based generation model applied when generating the PET-MRI fusion image in the storage unit 620, and temporarily or permanently stores data processed by the processor 630. The storage unit 620 may include a volatile storage medium or a non-volatile storage medium, but the scope of the present invention is not limited thereto.

In addition, when the PET-MRI fusion image generating apparatus 600 generates the PET-MRI fusion image for the sagittal plane including a partial region of interest of the MRI image, for example, the vertebral body, as described with reference to FIGS. 4 and 5, the storage unit 620 may store the AI model applied when extracting the sagittal plane including the vertebral body.

The PET-MRI fusion image generation method proposed in the present invention may be implemented in the form of program instructions that may be executed through various computer components and recorded in a computer-readable recording medium. The computer-readable recording medium may include program instructions, data files, data structures, and the like alone or in combination.

The program instructions recorded in the computer-readable recording medium may be specially designed and configured for the present invention or may be known to and used by those skilled in the field of computer software.

Examples of the computer-readable recording medium include a magnetic medium such as a hard disk, a floppy disk, and a magnetic tape, an optical recording medium such as a CD-ROM and a DVD, a magneto-optical medium such as a floptical disk, and a hardware device specially configured to store and execute program instructions such as a ROM, a RAM, a flash memory, and the like.

Examples of program instructions include not only machine language codes such as those generated by a compiler, but also high-level language codes that may be executed by a computer using an interpreter or the like. The hardware device may be configured to operate as one or more software modules to perform processing according to the present invention, and vice versa.

Although various embodiments of the present invention have been illustrated and described above, the present invention is not limited to the specific embodiments described above, and various modifications can be made by a person skilled in the art to which the present invention belongs without departing from the gist of the present invention claimed in the claims, and such modifications should not be individually understood from the technical spirit or the prospect of the present invention.

### Description of Reference Numerals

502: Vertebral body
504: Sagittal plane image on the left and right outer sides
506: Sagittal plane image involving vertebral body
508: MRI image
600: PET-MRI fusion image generating apparatus

## Claims

1. A method of generating a PET (Positron Emission Tomography)-MRI (Magnetic Resonance Imaging) fusion image using artificial intelligence, the method comprising:
when an ID (identifier) is input, calling MRI images corresponding to the patient ID and selecting one of the MRI images; and
applying a conditional GAN (Generative Adversarial Networks)-based generation model trained to derive a PET-MRI fusion image at the same anatomical position as the MRI image from the MRI image to generate a PET-MRI fusion image at the same anatomical position as the selected MRI image.

2. The method of claim 1, further comprising:
extracting a sagittal plane including a vertebral body from the selected MRI image; and
generating a PET-MRI fusion image having the same anatomical position as the extracted MRI image by applying the conditional GAN-based generation model.

3. The method of claim 2, wherein the extracting the sagittal plane including the vertebral body either (i) directly designated by the user, or (ii) automatically through repeated machine learning of an artificial intelligence model trained to extract the sagittal plane including the vertebral body from the MRI image.

4. The method of claim 1, wherein the selected MRI image is a T2 Fat Saturation (T2FS) sequence MRI image designed for fat attenuation.

5. The method of claim 1, wherein the conditional GAN-based generation model is operated by a generator and a discriminator, wherein the generator learns an MRI image and a PET-MRI fusion image of the same anatomical position obtained from an integrated PET-MRI apparatus to generate a PET-MRI fusion image of the same anatomical position as the selected MRI image, wherein the discriminator compares the generated PET-MRI fusion image with an actual PET-MRI fusion image obtained from the integrated PET-MRI apparatus, feeds back an error value between the comparison images to the generator, and wherein the generator generates a PET-MRI fusion image of the same anatomical position as the selected MRI image by reflecting the error value.

6. An apparatus for generating a PET (Positron Emission Tomography)-MRI (Magnetic Resonance Imaging) fusion image using artificial intelligence, the apparatus comprising: an input/output unit configured to, when an ID (identifier) is input, call MRI images corresponding to the patient ID and select one of the MRI images; a storage unit configured to store a GAN (Generative Adversarial Networks)-based generation model trained to derive a PET-MRI fusion image at the same anatomical position as the MRI image from the MRI image; and a processor configured to apply the conditional GAN-based generation model to generate a PET-MRI fusion image at the same anatomical position as the selected MRI image.

7. The PET-MRI fusion image generating apparatus of claim 6, wherein the processor is further configured to extract a sagittal plane including a vertebral body from the selected MRI image, and generate a PET-MRI fusion image having the same anatomical position as the extracted MRI image by applying the conditional GAN-based generation model.

8. The apparatus of claim 7, wherein the processor is configured to automatically extract the sagittal plane including the vertebral body through repeated machine learning of an artificial intelligence model trained to extract the sagittal plane including the vertebral body directly designated by a user or to extract the sagittal plane including the vertebral body from an MRI image.

9. The apparatus of claim 6, wherein the selected MRI image is a T2 Fat Saturation (T2FS) sequence MRI image designed for fat attenuation.

10. The PET-MRI fusion image generating apparatus of claim 6, wherein the conditional GAN-based generation model is operated by a generator and a discriminator, the generator learns an MRI image and a PET-MRI fusion image of the same anatomical position obtained from an integrated PET-MRI apparatus to generate a PET-MRI fusion image of the same anatomical position as the selected MRI image, the discriminator compares the generated PET-MRI fusion image with an actual PET-MRI fusion image obtained from the integrated PET-MRI apparatus, feeds back an error value between the comparison images to the generator, and the generator generates a PET-MRI fusion image of the same anatomical position as the selected MRI image by reflecting the error value.
